# EUROPÄISCHE PATENTSCHRIFT

(11) **EP 3 865 605 B1**
(45) Veröffentlichungstag und Bekanntmachung des Hinweises auf die Patenterteilung: **30.11.2022**
(21) Anmeldenummer: 21157625.1
(22) Anmeldetag: 17.02.2021
(51) Int. Cl.: E04C 5/01, C23F 13/20, C23F 13/04, G01N 27/02, G01N 33/38, G01N 17/02

(54) **SYSTEM FÜR DEN KATHODISCHEN KORROSIONSSCHUTZ, AKTIVVERTEILER UND WANDLERKNOTEN FÜR DAS SYSTEM**
CATHODIC CORROSION PROTECTION SYSTEM, ACTIVE MANIFOLD AND CONVERTER NODE FOR THE SYSTEM
SYSTÈME POUR LA PROTECTION CATHODIQUE CONTRE LA CORROSION, DISTRIBUTEUR ACTIF ET NOEUD DE CONVERTISSEUR POUR LE SYSTÈME

(30) Priorität: 17.02.2020 DE 102020104109
(43) Veröffentlichungstag der Anmeldung: 18.08.2021
(73) Patentinhaber: Geiger Bauwerksanierung GmbH & Co. KG, 87561 Oberstdorf (DE)
(72) Erfinder: Goeppel, Stefan, 88453 Erolzheim (DE)
(74) Vertreter: Canzler & Bergmeier Patentanwälte Partnerschaft mbB

(56) Entgegenhaltungen:
- EP-A1- 2 040 470
- EP-A2- 2 280 493
- WO-A1-93/11279
- AU-B2- 652 634
- DE-A1- 10 249 254
- DE-A1- 10 321 652
- DE-A1- 19 513 328
- US-A1- 2007 158 184
- US-A1- 2010 027 235

## Beschreibung

Die vorliegende Erfindung betrifft ein System für den kathodischen Korrosionsschutz eines Bauwerks mit wenigstens einem Versorgungselektrodenpaar zum Beaufschlagen einer Bewehrung des Bauwerks mit einer Spannung und wenigstens einer Referenzelektrode zur Messung eines elektrischen Potentials im Beton, wobei das Versorgungselektrodenpaar und die Referenzelektrode in einem Netzwerk angeordnet sind. Das Netzwerk umfasst weiterhin eine zentrale Schalteinheit, eine Verkabelung und wenigstens einen Verteiler. Weiterhin umfasst die Erfindung einen Aktivverteiler und einen Wandlerknoten für ein derartiges System.

Mit Stahl bewehrter Beton ist als Baustoff unverzichtbar geworden. Allerdings kann eine Korrosion der Bewehrung starke Schäden bis hin zu einer Gefährdung der strukturellen Integrität bei aus Stahlbeton hergestellten Bauwerken verursachen. Zum Schutz der Bewehrung vor anfänglicher Korrosion oder weiterer Korrosion bei bereits vorhandenen Schäden, ist es bekannt, die Bewehrung mit einer elektrischen Spannung zu beaufschlagen. Die Bewehrung wird dabei mit dem Minuspol einer Gleichstromquelle verbunden und damit zu einer Kathode. Gleichzeitig werden an oder in dem bewehrten Beton metallische Strukturen vorgesehen, die mit dem Pluspol der Gleichstromquelle verbunden werden und damit als Anode dienen. Durch die Veränderung des elektrischen Potentials wird eine Korrosion der Bewehrung dauerhaft verhindert. Auf diese Weise können Bauwerke mit dem Einsatz einer geringen elektrischen Leistung dauerhaft vor Schäden durch Korrosion der Bewehrung geschützt werden. Weiterhin besteht ein Interesse die Funktionsfähigkeit eines solchen sogenannten kathodischen Korrosionsschutzes zu überwachen. Diese Überwachung kann mit Referenzelektroden durchgeführt werden, die an bestimmten Punkten des Bauwerks im Beton vorgesehen sind und das elektrische Potential im Beton überwachen. In der Regel werden der kathodische Korrosionsschutz und die Überwachung in einem gemeinsamen System kombiniert.

Ein derartiges System ist beispielsweise aus der WO 1993/11279 A1 bekannt. Das System weist ein verzweigtes Netzwerk mit einem primären Netzteil und einem Computer auf. Über eine zweiadrige Verbindungsleitung des Netzwerks werden einerseits die Bewehrung und andererseits die Anoden mit einer elektrischen Spannung beaufschlagt. Das Netzwerk weist weiterhin Verteilerknoten auf, die mit mehreren Elektroden verbunden sein können. Nachteilig bei dem bekannten System ist, dass einerseits die Länge der Verbindungsleitung und andererseits die Anzahl der Teilnehmer in dem Netzwerk durch den in der Leitung stattfindenden Spannungsabfall stark begrenzt ist. Spätestens bei einem größeren Bauwerk, in dem längere Verbindungsleitungen notwendig wären, stößt ein derartiges System an seine Grenzen. Andererseits ist die Datenübertragung in der Versorgungsleitung fehleranfällig. Darüber hinaus wäre eine Überwachung des Systems mittels dedizierter Referenzelektroden für eine unabhängige Überprüfung wünschenswert.

Weitere ähnliche Systeme offenbaren beispielsweise die US 2007/0158184 A1 und die AU 652 634 B2. Ein System zur Überwachung eines Korrosionsschutzsystems ist beispielsweise in der US 2010/0027235 A1 offenbart. Die DE 102 49 254 A1 offenbart einen aktiven Verteiler in einem Kommunikationsnetzwerk, die EP 2 040 470 A1 einen programmierbaren Aktivverteiler für eine Fernseh-Kopfstelle. Die EP 2 280 493 A2 offenbart ein sicherheitsbezogenes Kommunikationsverfahren auf Energieversorgungsleitungen und ein dazugehöriges Netz. Die DE 103 21 652 A1 offenbart ein modulares Datenerfassungs- und Übertragungssystem. Die DE 195 13 328 A1 offenbart außerdem eine Anordnung zur Verarbeitung und Anzeige von Informationen.

Aufgabe der vorliegenden Erfindung ist es, die bekannten Systeme für den kathodischen Korrosionsschutz im Hinblick auf die genannten Nachteile zu verbessern.

Die Aufgabe wird gelöst durch ein System mit den Merkmalen des Anspruchs 1.

Das erfindungsgemäße System für den kathodischen Korrosionsschutz eines Bauwerks aus bewehrtem Beton weist wenigstens ein Versorgungselektrodenpaar zum Beaufschlagen einer Bewehrung des Bauwerks mit einer Spannung auf. Weiterhin weist das System wenigstens eine Referenzelektrode zur Messung eines elektrischen Potenzials im Beton auf, wobei das Versorgungselektrodenpaar und die Referenzelektrode in einem Netzwerk angeordnet sind. Vorzugsweise umfasst das System eine Vielzahl an Versorgungselektrodenpaaren und/oder Referenzelektroden. Das Netzwerk umfasst weiterhin eine zentrale Schalteinheit, eine Verkabelung und wenigstens einen Verteiler. Die Verkabelung weist einen Kabelstrang auf. Ein System mit diesen Merkmalen ist beispielsweise aus der WO 1993/11279 A1 bekannt. Erfindungsgemäß wird vorgeschlagen, dass der Kabelstrang eine Versorgungsleitung und eine von der Versorgungsleitung getrennte Datenleitung umfasst. Die Versorgungsleitung dient zur Stromversorgung des Versorgungselektrodenpaars. Die Datenleitung dient zur Datenübertragung, insbesondere zwischen der Referenzelektrode und der zentralen Schalteinheit. Außerdem ist das Versorgungselektrodenpaar über einen Wandlerknoten mit dem Netzwerk verbunden, wobei der Wandlerknoten wenigstens einen wenigstens zweistufigen Spannungswandler aufweist.

Durch die Trennung von Daten- und Versorgungsleitung kann beispielsweise an die Versorgungsleitung eine höhere Spannung angelegt werden, ohne dass die datenverarbeitenden Elemente des Netzwerks höheren Belastungen ausgesetzt werden. Durch eine höhere Spannung können dem Netzwerk mehr Teilnehmer hinzugefügt und eine höhere gesamte Verkabelungslänge des Netzwerks erreicht werden. Weiterhin ist die Datenübertragung weniger störanfällig und es ist beispielsweise keine Modellierung der Daten notwendig.

Für den kathodischen Korrosionsschutz des Bauwerks ist das Versorgungselektrodenpaar insbesondere einerseits mit der Bewehrung verbunden und andererseits mit einer Anodenstruktur. Die Anodenstruktur kann beispielsweise in Form eines Metallbands oder Metallnetzes vorliegen. Ebenfalls denkbar sind einzelne Metallstifte. Das Metall der Anodenstruktur kann beispielsweise unedler sein als das im Stahl der Bewehrung enthaltene Eisen. Unedler bedeutet beispielsweise, dass die Elektronegativität des Metalls der Anodenstruktur geringer ist als die des Eisens. Mögliche Metalle umfassen beispielsweise Titan oder Magnesium. Die Anodenstruktur kann von außen in Kontakt mit dem Beton gebracht werden und insbesondere anschließend über einen Ankopplungsmörtel dauerhaft mit dem Beton verbunden werden oder beispielsweise durch Bohrungen in den Beton eingebracht werden. Es ist ebenfalls denkbar, dass eine Anodenstruktur bereits bei der Herstellung des bewehrten Betons bzw. des Bauwerks vorgesehen wird. Bei einem größeren Bauwerk liegen vorzugsweise mehrere Versorgungselektrodenpaare bzw. eine Vielzahl von Versorgungselektrodenpaaren vor. Auf diese Weise können insbesondere nicht miteinander verbundene Bereiche der Bewehrung gemeinsam geschützt werden. Insbesondere werden die Bewehrung und die Anodenstruktur mit einer Gleichspannung beaufschlagt. Die Bewehrung ist insbesondere mit einem Minuspol des Versorgungselektrodenpaars verbunden.

Die Referenzelektrode dient der unabhängigen Überprüfung der Funktion des kathodischen Korrosionsschutzes. Die Referenzelektrode ist beispielsweise mittels einer Bohrung in den Beton eingelassen. Insbesondere können Referenzelektroden an statisch wichtigen Stellen des Bauwerks vorgesehen sein. Beispielswiese im Bereich tragender Säulen. Bei einem größeren Bauwerk ist insbesondere eine Vielzahl an Referenzelektroden vorgesehen. Den Gegenpol zur Referenzelektrode bildet insbesondere die Bewehrung. Die Referenzelektrode ist beispielsweise als Mangan-Mangandioxid-Elektrode oder als Silber-Silberchlorid-Elektrode ausgebildet.

Das Netzwerk ist beispielsweise als Bus und insbesondere als serieller oder paralleler Bus ausgebildet. Die zentrale Schalteinheit weist beispielsweise einen Zentralknoten des Netzwerks bzw. des Buses auf. Weiterhin kann die zentrale Schalteinheit beispielsweise ein zentrales Netzteil, einen Computer insbesondere mit einem nichtflüchtigen Speicher zum Speichern gesammelter Daten, Sicherungen und/oder ein Benutzerinterface aufweisen. Das Benutzerinterface dient beispielsweise einer Abfrage der Messdaten der Referenzelektroden. Die zentrale Schalteinheit ist insbesondere an einem gut zugänglichen Ort des Bauwerks vorgesehen. Weiterhin ist die zentrale Schalteinheit vorzugsweise mit dem öffentlichen Stromnetz verbunden. Die zentrale Schalteinheit kann beispielsweise eine Batterie aufweisen, zum Schutz eines Ausfalls der öffentlichen Stromversorgung.

Der Verteiler weist im einfachsten Fall beispielsweise eine Eingangsleitung und zwei Ausgangsleitungen auf, und verzweigt so sowohl die Versorgungsleitung als auch die Datenleitung des Netzwerks. Durch eine Verzweigung des Netzwerks lassen sich unnötig lange Verkabelungen vermeiden. Das zu schützende Bauwerk kann beispielsweise eine Brücke, ein industrielles Gebäude, wie insbesondere ein Parkhaus oder ein Hochhaus sein.

Bei einer Umwandlung der Spannung im Wandlerknoten in mehreren kleinen Schritten treten weniger Leistungsverluste auf als bei einer Umwandlung in wenigen großen Schritten. Auch eine Erwärmung des Wandlerknotens wird hierdurch verringert. Beispielsweise kann ein erster Spannungswandler oder eine erste Wandlerstufe eine feste Ausgangsspannung, ein zweiter Spannungswandler oder eine zweite Wandlerstufe ein Regelungsintervall der Ausgangsspannung von 0,1 V und ein dritter Spannungswandler oder eine dritte Wandlerstufe ein Regelungsintervall der Ausgangsspannung von 1 mV aufweisen. Der Wandlerknoten kann beispielsweise eine galvanische Trennung, insbesondere in Form von zwei Spulen mit einem Luftspalt, aufweisen. Weiterhin ist es besonders vorteilhaft, wenn der Wandlerknoten wenigstens zwei und insbesondere drei Spannungswandler aufweist. Außerdem ist es vorteilhaft, wenn der Wandlerknoten wenigstens einen wenigstens dreistufigen Spannungswandler aufweist.

In einer vorteilhaften Weiterbildung des Systems umfasst die Versorgungsleitung zwei Adern und die Datenleitung umfasst wenigstens zwei Adern. Auf diese Weise lässt sich an der Versorgungsleitung und der Datenleitung unabhängig voneinander Gleichstrom anlegen. Vorzugsweise ist die Spannung an der Versorgungsleitung größer als die Spannung an der Datenleitung. Die Adern sind beispielsweise als Kupferdrähte ausgebildet. Zumindest die Datenleitung weist insbesondere eine Abschirmung auf, um eine Störanfälligkeit zu reduzieren. Es ist insbesondere denkbar, dass die Datenleitung vier Adern aufweist, um den Datendurchsatz zu erhöhen oder um die Störungsanfälligkeit der Datenübertragung weiter zu reduzieren. Selbstverständlich sind die einzelnen Adern voneinander isoliert, wobei die Versorgungsleitung und die Datenleitung aber beispielsweise in einer gemeinsamen Isolierung angeordnet sind.

Vorteilhafterweise liegt an der Versorgungsleitung eine größere Spannung an als diejenige, die für den kathodischen Korrosionsschutz bei den einzelnen Versorgungselektrodenpaaren notwendig ist. Einerseits liegt bei einer größeren Spannung in der Versorgungsleitung ein niedrigerer Leistungsverlust im Übertragungsweg vor und es kann auf diese Weise eine größere Anzahl an Versorgungselektrodenpaaren mit elektrischer Leistung versorgt werden. Der Wandlerknoten dient in erster Linie dazu, die höhere Spannung in der Versorgungsleitung für die einzelnen Versorgungselektrodenpaare auf einen niedrigeren Wert herunterzutransformieren.

Der Wandlerknoten kann gleichzeitig beispielsweise einer Steuerung oder Regelung der an dem Versorgungselektrodenpaar anliegenden Spannung dienen. Der Wandlerknoten kann beispielsweise die anliegende Spannung in Abhängigkeit der Messwerte der Referenzelektroden steuern. Hierzu weist der Wandlerknoten beispielsweise einen Mikrocontroller auf. Auch kann der Wandlerknoten insbesondere die an dem Versorgungselektrodenpaar anliegende Spannung an die zentrale Schalteinheit zurückmelden, bzw. von dieser Anweisungen über die anzulegende Spannung bzw. die anzulegende Stromstärke erhalten.

Weiterhin stellt es einen Vorteil dar, wenn die Referenzelektrode über einen Messknoten mit dem Netzwerk verbunden ist, wobei der Messknoten wenigstens einen Analog-Digital-Wandler und/oder Mikrocontroller umfasst. Der Messknoten übernimmt beispielsweise die Kommunikation der Messdaten der Referenzelektrode an die zentrale Schalteinheit. Die Messdaten werden vorzugsweise in digitaler Form über die Datenleitung kommuniziert, wobei die von der Referenzelektrode gemessenen analogen Daten zunächst insbesondere vom Analog-Digital-Wandler digitalisiert werden. Insbesondere kann der Mikrocontroller den Analog-Digital-Wandler umfassen.

Es ist denkbar, dass der Messknoten ausschließlich oder zusätzlich mit anderen Messelementen verbunden ist. Diese Messelemente können beispielsweise Strommesser, Thermometer, pH-Meter oder Feuchtigkeitsmesser umfassen, die insbesondere am oder im Beton angeordnet sind. Die von diesen Messelementen gelieferten Daten können wichtige Informationen über den Zustand des Bauwerks liefern. Diese Informationen werden insbesondere in der zentralen Schalteinheit zusammengetragen und beispielswiese miteinander korreliert.

Es ist vorteilhaft, wenn eine an der Versorgungsleitung anliegende Spannung größer oder gleich 24 V, 48 V oder 60 V ist. Wie bereits beschrieben führt eine höhere Spannung zu niedrigeren Verlusten bei der Übertragung. Dies ermöglicht eine größere Zahl an Teilnehmern im Netzwerk und eine größere maximale Verkabelungslänge. Andererseits können insbesondere Halbleiterelemente mit großen Spannungen nicht umgehen. Daher stellen die genannten Spannungen von 24 V, 48 V oder 60 V einen vorteilhaften Kompromiss für das erfindungsgemäße System dar. Außerdem sind die genannten Spannungen bei geringen Stromstärken für Menschen ungefährlich. Besonders bevorzugt beträgt die Spannung an der Versorgungsleitung 48 V.

Für eine Verzweigung des Netzwerks ist es besonders vorteilhaft, wenn der Verteiler als Passivverteiler oder Aktivverteiler ausgebildet ist, wobei der Aktivverteiler insbesondere eine externe Stromversorgung und/oder eine mit der zentralen Schalteinheit verbundene zusätzliche Versorgungsleitung und/oder ein Signalaufbereitungsmodul aufweist. Wie vorher bereits beschrieben kann mit einer Verzweigung des Netzwerks unnötige zusätzliche Verkabelung eingespart werden, und es können auf einfache Weise unterschiedliche Bereiche eines Bauwerks mit einem System geschützt und überwacht werden. Der Passivverteiler teilt hierbei eine Eingangsleitung in zwei Ausgangsleitungen auf, wobei der Passivverteiler keine zusätzlichen Funktionen, wie beispielsweise Signalaufbereitung oder aktive Verteilung von Datenpaketen umfasst.

Der Aktivverteiler versorgt das Netzwerk mit zusätzlicher elektrischer Leistung und/oder bereitet die im Netzwerk übertragenen Daten auf. Falls der Aktivverteiler die externe Stromversorgung oder die mit der zentralen Schalteinheit verbundene zusätzliche Versorgungsleitung aufweist, kann er beispielsweise die volle anfängliche Spannung in der Versorgungsleitung wiederherstellen und Verluste durch die Übertragung und Abnehmer ausgleichen. Die maximale Größe des Netzwerks wird so erhöht. Die externe Stromversorgung ist insbesondere mit dem öffentlichen Stromnetz verbunden.

Das Signalaufbereitungsmodul kann beispielsweise den vollen anfänglichen Spannungshub eines übertragenen Bits wiederherstellen und damit die überbrückbare Gesamtlänge für die Daten deutlich erhöhen. Die hierzu nötige elektrische Leistung kann der Aktivknoten aus der Versorgungsleitung des Netzwerks, der externen Stromversorgung und/oder der zusätzlichen Versorgungsleitung beziehen. Das Signalaufbereitungsmodul kann beispielweise als Regenerator oder Verstärker ausgebildet sein.

Vorteilhafterweise weist der Aktivverteiler einen Mikrocontroller und/oder wenigstens zwei, vorzugsweise wenigstens vier, Ausgangsleitungen auf. Hierdurch können das Netzwerk weiter verzweigt und eventuell zusätzliche Teilnehmer dem Netzwerk hinzugefügt werden. Eine eventuelle externe Stromversorgung oder zusätzliche Versorgungsleitung ermöglicht dem Aktivverteiler beispielsweise auf allen Ausgangsleitungen die volle ursprüngliche Spannung und die maximale Qualität der übertragenen Daten bereitzustellen. Der Mikrocontroller und/oder ein zusätzliches Netzwerkmodul können beispielsweise dem aktiven Verteilen von Datenpaketen auf die Ausgangsleitungen dienen. Das bereits beschriebene Signalaufbereitungsmodul kann beispielsweise Bestandteil des Mikrocontrollers sein.

Beispielsweise können mittels einer Ausgangsleitung der zentralen Schalteinheit an einem Kabelstrang 256 Teilnehmer mit dem Netzwerk verbunden werden, wobei mit Teilnehmer jeder Verteiler, Wandlerknoten, Messknoten und/oder Aktivverteiler gemeint ist. Durch die zusätzliche Energieversorgung und Datenaufbereitung des Aktivverteilers können beispielweise an jeder Ausgangsleitung des Aktivverteilers 256 weitere Teilnehmer mit dem Netzwerk verbunden werden. Auf diese Weise lassen sich auch sehr große Bauwerke effizient schützen und überwachen.

Zusätzlich ist es von Vorteil, wenn die zentrale Schalteinheit und/oder einer der wenigstens einen Aktivverteiler ein Modul zur kabellosen Datenübertragung aufweist. Eventuell kann auf diese Weise eine Verkabelung zwischen dem Aktivverteiler und der zentralen Schalteinheit oder zwischen zwei Aktivverteilern eingespart werden und die Datenübertragung rein kabellos erfolgen. Falls ein Aktivverteiler nicht durch die Verkabelung mit der zentralen Schalteinheit verbunden ist, ist eine externe Stromversorgung für diesen Aktivverteiler zwingend notwendig. Es ist denkbar, dass alle Aktivverteiler ein Modul zur kabellosen Datenübertragung aufweisen. Die Datenübertragung kann beispielsweise über einen WLAN- oder Mobilfunkstandard erfolgen.

Besonders vorteilhaft ist es in diesem Zusammenhang, wenn das Modul zur kabellosen Datenübertragung zur Verwendung von Kanalbreiten im Bereich von 180 kHz ausgebildet ist. Datenübertragungen mit diesen Kanalbreiten weisen eine vergleichsweise niedrige Datenübertragungsrate auf, eigenen sich aber besonders für die Datenübertragung innerhalb von Bauwerken mit einer Vielzahl von Hindernissen, wie Wände oder Stützen, die eine breitbandige Datenübertragung erschweren. Insbesondere ist das Modul zur kabellosen Datenübertragung ausgebildet nach dem "NarrowBand IoT"-Standard zu arbeiten, der die obigen Kanalbreiten verwendet.

In einer weiteren vorteilhaften Ausgestaltung des Systems weist die zentrale Schalteinheit und/oder einer der wenigstens einen Aktivverteiler ein Powerlinemodul zur Datenübertragung über das allgemeine Stromnetz auf. Das Powerlinemodul moduliert die zu übertragenden Daten auf den Wechselstrom des öffentlichen Stromnetzes auf, womit bei einer externen Stromversorgung des Aktivverteilers wiederum eine Verkabelung zwischen dem Aktivverteiler und der zentralen Schalteinheit oder zwischen zwei Aktivverteilern eingespart werden kann.

Außerdem ist es von Vorteil, wenn die zentrale Schalteinheit und/oder einer der wenigstens einen Aktivverteiler ein Modem zur Datenübertragung über das Internet aufweist. Einerseits können Daten hierdurch über ein öffentliches Mobilfunknetz übertragen werden. Andererseits können die Daten aber ebenfalls über das kabelgebundene Telefonnetz bzw. Datennetz übertragen werden. Die Daten können einerseits zwischen den Aktivknoten und/oder der zentralen Schalteinheit ein und desselben Systems übertragen werden. Andererseits können die Daten über das Modem auch zwischen verschiedenen Systemen übertragen werden oder beispielsweise an einen Zentralserver eines Betreibers des Systems.

Insbesondere ist es vorteilhaft, wenn eine Gesamtanzahl der Passivverteiler, Wandlerknoten, Messknoten und/oder Aktivverteiler in dem Netzwerk größer als 100 und insbesondere größer als 256 ist. Eine größere Gesamtzahl an Teilnehmern im Netzwerk erlaubt beispielsweise eine sehr gute Verteilung der Versorgungselektrodenpaare und damit einen umfassenden Korrosionsschutz des Bauwerks. Weiterhin ist eine fein verteilte Überwachung des kathodischen Korrosionsschutzes durch die Messknoten möglich. Insbesondere größere Bauwerke lassen sich auf diese Weise effizient schützen und überwachen. Allen Teilnehmern des Netzwerks kann beispielsweise eine individuelle Hardwareadresse zugeordnet sein. Die Hardwareadresse kann beispielsweise aus 4 Stellen bestehen, womit insgesamt 99 mal 99 Teilnehmer des Netzwerks jeweils mit einer individuellen Adresse möglich sind. Individuelle Hardwareadressen ermöglichen es beispielsweise einzelne Teilnehmer des Netzwerks problemlos auszutauschen, oder das Netzwerk problemlos um weitere Teilnehmer zu erweitern.

Ein weiterer Vorteil zeigt sich, wenn jeder Wandlerknoten, Messknoten und/oder Aktivverteiler ein Speichermodul aufweist. Das Speichermodul bzw. die Speichermodule können beispielsweise einer dezentralen Speicherung und/oder Zwischenspeicherung der von dem jeweiligen Teilnehmer übertragenen Daten dienen. Dies führt zu einer Redundanz der Daten zwischen den Netzwerkteilnehmern und der zentralen Schalteinheit und kann einem Datenverlust vorbeugen. Die Daten können zusätzlich oder alternativ beispielsweise durch das oben beschriebene Modem in einem Cloudspeicher abgelegt werden.

Bei dem Aktivverteiler des Systems für den kathodischen Korrosionsschutz eines Bauwerks aus bewehrtem Beton, das gemäß der vorangegangenen Beschreibung ausgebildet ist, wird vorgeschlagen, dass der Aktivverteiler einen Mikrocontroller und/oder wenigstens zwei, vorzugsweise wenigstens vier, Ausgangsleitungen aufweist.

Wie bereits beschrieben kann der Aktivverteiler die Gesamtgröße eines Netzwerks des Systems für den kathodischen Korrosionsschutz deutlich erhöhen und damit beispielswiese auch größere Bauwerke effizient schützen und überwachen.

Der Aktivverteiler kann eine externe Stromversorgung und/oder eine mit einer zentralen Schalteinheit des Systems verbundene zusätzliche Versorgungsleitung und/oder ein Signalaufbereitungsmodul aufweisen. Weiterhin kann der Aktivverteiler ein Modul zur kabellosen Datenübertragung, das insbesondere ausgebildet ist den "NarrowBand loT" Standard zu unterstützen, ein Powerlinemodul und/oder ein Modem aufweisen.

Für den Wandlerknoten des Systems für den kathodischen Korrosionsschutz eines Bauwerks aus bewehrtem Beton, das gemäß der vorangegangenen Beschreibung ausgebildet ist, mit einem ersten Spannungswandler, wird vorgeschlagen, dass der erste Spannungswandler wenigstens zweistufig und vorzugsweise dreistufig ausgebildet ist.

Wie bereits beschrieben verringert eine mehrstufige Umwandlung der Spannung Verluste der elektrischen Leistung. Beispielsweise kann ein erster Spannungswandler oder eine erste Wandlerstufe eine feste Ausgangsspannung, ein zweiter Spannungswandler oder eine zweite Wandlerstufe ein Regelungsintervall der Ausgangsspannung von 0,1 V und ein dritter Spannungswandler oder eine dritte Wandlerstufe ein Regelungsintervall der Ausgangsspannung von 1 mV aufweisen. Der Wandlerknoten kann beispielsweise eine galvanische Trennung, insbesondere in Form von zwei Spulen mit einem Luftspalt, aufweisen.

Weitere Vorteile der Erfindung sind in den nachfolgenden Ausführungsbeispielen beschrieben. Es zeigt:
- **Figur 1**: eine schematische Übersicht des erfindungsgemäßen Systems und
- **Figur 2**: eine schematische Ansicht eines Bauwerks mit dem System.

Bei der nachfolgenden Beschreibung der Figuren werden für in den verschiedenen Figuren jeweils identische und/oder zumindest vergleichbare Merkmale gleiche Bezugszeichen verwendet. Die einzelnen Merkmale, deren Ausgestaltung und/oder Wirkweise werden meist nur bei ihrer ersten Erwähnung ausführlich erläutert. Werden einzelne Merkmale nicht nochmals detailliert erläutert, so entspricht deren Ausgestaltung und/oder Wirkweise der Ausgestaltung und Wirkweise der bereits beschriebenen gleichwirkenden oder gleichnamigen Merkmale.

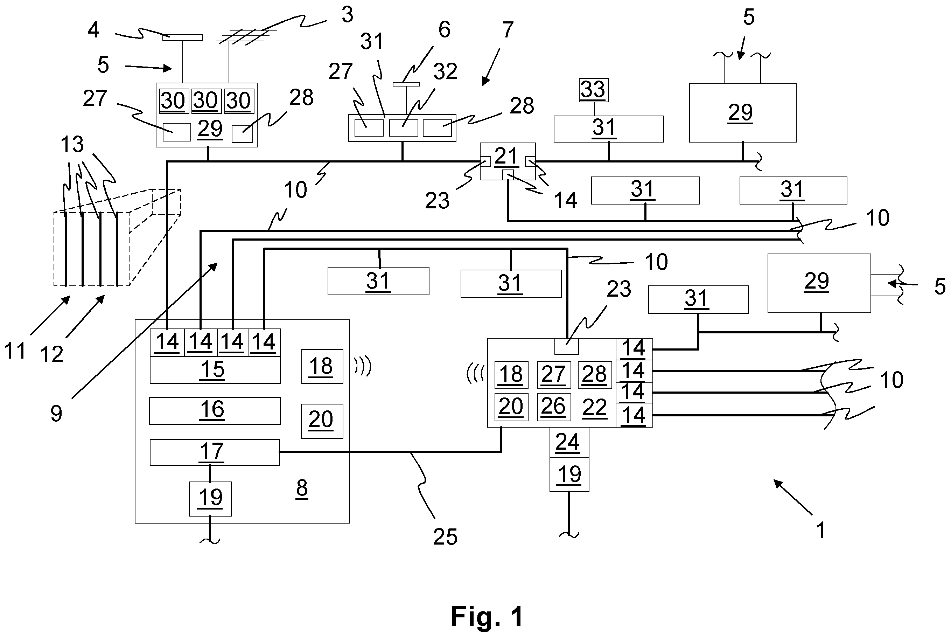

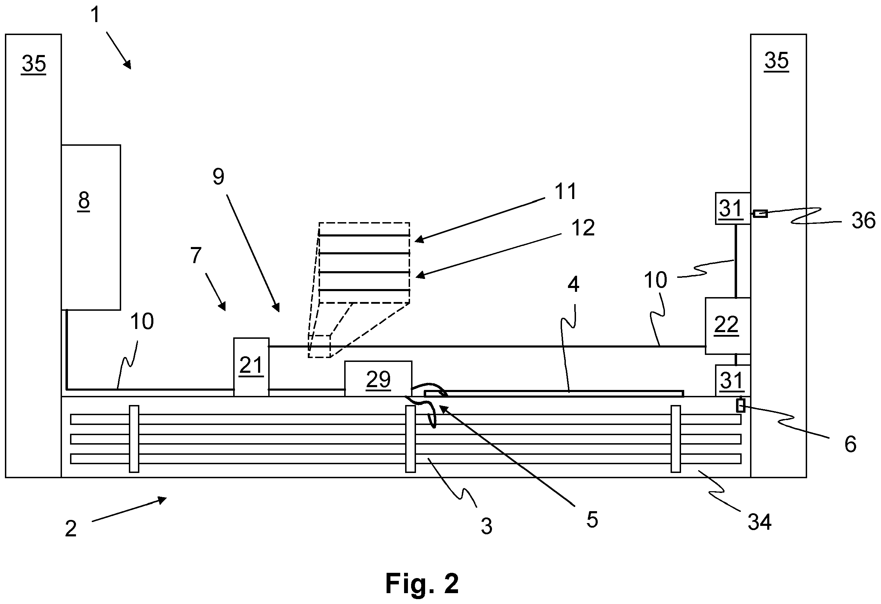

Figur 1 zeigt ein Schema eines Systems 1 für den kathodischen Korrosionsschutz eines Bauwerks 2 (siehe Figur 2) aus bewehrtem Beton. Der kathodisehe Korrosionsschutz wird durch das Anlegen einer Spannung zwischen einer Bewehrung 3 des Bauwerks 2 und einer Anode 4 bewirkt, wobei hierdurch die Oxidation der Bewehrung 3 verhindert wird. Besagte Spannung wird von einem Versorgungselektrodenpaar 5 an die Bewehrung 3 und die Anode 4 übertragen. Das System 1 gewährleistet die Spannungsversorgung der Versorgungselektrodenpaare 5 und die Überwachung der Funktion des kathodischen Korrosionsschutzes. Versorgungselektrodenpaare 5 können potentiell an vielen Stellen eines Bauwerks 2 platziert sein. Auch ist eine Überwachung an vielen Stellen vorteilhaft.

Die Überwachung wird durch Referenzelektroden 6 gewährleistet, die das elektrische Potential im Beton messen. Zur gemeinsamen Verwaltung der Versorgungselektrodenpaare 5 und der Referenzelektroden 6 sind diese in einem Netzwerk 7 angeordnet. Das Netzwerk 7 umfasst eine zentrale Schalteinheit 8, von der eine Verkabelung 9 ausgeht. Die Verkabelung 9 umfasst mehrere Kabelstränge 10, die einzelne Teilnehmer des Netzwerks 7 miteinander verbinden. Das System 1 zeichnet sich unter anderem dadurch aus, dass die Kabelstränge 10 eine Versorgungsleitung 11 und eine von der Versorgungsleitung 11 getrennte Datenleitung 12 aufweisen. Der Aufbau des Kabelstrangs 10 ist in der angedeuteten Vergrößerung näher dargestellt. In diesem Beispiel weisen die Versorgungsleitung 11 und die Datenleitung 12 jeweils zwei Adern 13 auf. Eine an der Versorgungsleitung 11 anliegende Gleichspannung ist insbesondere größer als eine an der Datenleitung 12 anliegende Gleichspannung.

In diesem Beispiel gehen von insgesamt vier Ausgangsleitungen 14 der zentralen Schalteinheit 8 vier Kabelstränge 10 ab. Die Ausgangsleitungen 14 der zentralen Schalteinheit 8 werden von einem Zentralknoten 15 verwaltet. Zur Datenverarbeitung weist die zentrale Schalteinheit 8 weiterhin einen Computer auf. Zur Energieversorgung weist die zentrale Schalteinheit 8 ein Netzteil 17 auf, das insbesondere mit einem öffentlichen Stromnetz verbunden ist. Die zentrale Schalteinheit 8 kann weiterhin ein Modul zur kabellosen Datenübertragung 18, ein Powerlinemodul 19 und/oder ein Modem 20 aufweisen. Das Modul zur kabellosen Datenübertragung 18 dient der Kommunikation mit anderen Bauteilen, die ebenfalls ein Modul zur kabellosen Datenübertragung 18 aufweisen, innerhalb des Bauwerks 2. Die Datenübertragung erfolgt hierbei vorzugsweise gemäß dem "NarrowBand IoT"-Standard. Das Powerlinemodul 19 dient der Datenübertragung über das öffentliche und/oder bauwerksinterne Wechselstromnetz zwischen weiteren Bauteilen, die ebenfalls mit dem Wechselstromnetz verbunden sind und ein Powerlinemodul 19 aufweisen. Das Powerlinemodul 19 moduliert die zu übertragenden Daten hierbei auf die periodische Spannungs- bzw. Stromkurve des Wechselstromnetzes auf und das empfangende Powerlinemodul 19 führt eine entsprechende Demodulation durch. Das Modem 20 dient einer Datenübertragung über das Internet. Hierfür kommt das Mobilfunknetz oder das kabelgebundene Telefon- bzw. Datennetz in Frage.

Das beispielhafte Netzwerk 7 verzweigt sich an mehreren Stellen, wobei das Netzwerk 7 vorliegend einen Passivverteiler 21 und einen Aktivverteiler 22 aufweist. Der Passivverteiler 21 weist eine Eingangsleitung 23 und zwei Ausgangsleitungen 14 auf, wobei ein Kabelstrang 10 zur Eingangsleitung 23 hinführt und je ein Kabelstrang 10 von jeder Ausgangsleitung 14 wegführt. An jedem Kabelstrang 10, der auf diese Weise von dem Passivverteiler 21 wegführt, können wieder mehrere Teilnehmer angeordnet sein.

Der Aktivverteiler 22 umfasst weitere Funktionen. Beispielswiese kann der Aktivverteiler 22 vier Ausgangsleitungen 14 aufweisen, von denen jeweils ein Kabelstrang 10 wegführt. Weiterhin kann der Aktivverteiler 22 eine externe Stromversorgung 24, eine mit der zentralen Schalteinheit 8 verbundene zusätzliche Versorgungsleitung 25 und/oder ein Signalaufbereitungsmodul 26 aufweisen. Diese Merkmale jeweils allein oder in beliebiger Kombination erlauben es dem Aktivverteiler 22 die Gesamtzahl der Teilnehmer im Netzwerk 7 sowie die maximale Länge der Verkabelung 9 zu erhöhen. Die externe Stromversorgung 24 ist insbesondere mit dem öffentlichen bzw. bauwerksinternen Wechselstromnetz verbunden. Beispielsweise zur Verteilung von Datenpaketen auf die Ausgangsleitungen 14 kann der Aktivverteiler 22 einen Mikrocontroller 27 aufweisen. Auch kann der Aktivverteiler 22 zum Zwischenspeichern der übertragenen Daten ein Speichermodul 28 aufweisen.

Der Aktivverteiler 22 kann zur Datenübertragung ein Modul zur kabellosen Datenübertragung 18, ein Powerlinemodul 19 und/oder ein Modem 20 aufweisen. Diese Merkmale können wie bei der zentralen Schalteinheit 8 ausgebildet sein. Falls der Aktivverteiler 22 eine vom Netzwerk 7 unabhängige Stromversorgung und ein Modul zur kabellosen Datenübertragung 18, ein Powerlinemodul 19 und/oder ein Modem 20 aufweist, sind eine Verbindung zum Netzwerk 7 mittels eines Kabelstrangs 10 und eine Eingangsleitung 23 nicht unbedingt erforderlich. Diese sind also im vorliegenden Beispiel als optional anzusehen.

Die weiteren Verzweigungen in den Kabelsträngen 10, an denen jeweils ein Teilnehmer angeordnet ist, dienen nur der Übersichtlichkeit der Darstellung.

Die Versorgungselektrodenpaare 5 sind jeweils über einen Wandlerknoten 29 mit dem Netzwerk 7 verbunden. Der Wandlerknoten 29 dient der Umwandlung einer hohen Spannung in der Versorgungsleitung 11 zu einer für den kathodischen Korrosionsschutz ausreichenden niedrigeren Spannung. Hierzu weist der Wandlerknoten 29 wenigstens einen Spannungswandler 30 auf, wobei der Wandlerknoten 29 auch zwei Spannungswandler 30, oder wie in diesem Beispiel drei Spannungswandler 30 aufweisen kann. Alternativ kann der Wandlerknoten 29 auch einen zwei- oder dreistufigen Spannungswandler 30 aufweisen. Der Übersicht halber ist nur einer der mehreren Wandlerknoten 29 dieses Ausführungsbeispiels detailliert dargestellt. Die Beschreibung trifft aber auf alle Wandlerknoten 29 des Systems 1 zu. Zur Datenverarbeitung kann der Wandlerknoten 29 weiterhin einen Mikrocontroller 27 aufweisen. Außerdem kann der Wandlerknoten 29 zur Datenspeicherung ein Speichermodul 28 aufweisen.

Die Referenzelektroden 6 (der Übersicht halber ist nur eine dargestellt) sind über Messknoten 31 mit dem Netzwerk 7 verbunden. Die Messknoten 31 dienen der Datenaufbereitung und Datenübertragung. Hierzu weisen die Messknoten 31 einen Mikrocontroller 27 und/oder einen Analog-Digital-Wandler 32 auf. Der Analog-Digital-Wandler 32 dient beispielsweise der Digitalisierung der analogen Messdaten der Referenzelektroden 6. Die Messknoten 31 können aber auch zusätzlich oder ausschließlich die Messdaten weiterer Messelemente, wie beispielswiese eines Temperaturfühlers 33, erfassen, gegebenenfalls digitalisieren und an die zentrale Schalteinheit 8 senden. Zur Speicherung der Daten kann der Messknoten 31 ein Speichermodul 28 aufweisen. Wie bei den Wandlerknoten 29 ist auch bei den Messknoten 31 nur einer detailliert dargestellt. Wie zuvor gilt die Beschreibung aber für alle Messknoten 31.

Figur 2 zeigt ein schematisches Bauwerk 2 mit einem erfindungsgemäßen System 1. Es sind eine Bodenplatte 34 und zwei Wände 35 des Bauwerks 2 dargestellt, wobei insbesondere die Bodenplatte 34 aus bewehrtem Beton hergestellt ist und eine Bewehrung 3 aufweist. Durch das System 1 wird die Bewehrung 3 vor Oxidation bzw. Korrosion geschützt. An einer der Wände 35 ist eine zentrale Schalteinheit 8 des Systems 1 angeordnet. Eine Verkabelung 9 mit mehreren Kabelsträngen 10 baut ein Netzwerk 7 auf, in dem ein Versorgungselektrodenpaar 5 und eine Referenzelektrode 6 angeordnet sind.

Das Versorgungselektrodenpaar 5 ist einerseits mit der Bewehrung 3 in der Bodenplatte 34 und andererseits mit einer Anode 4, die auf einer Oberfläche der Bodenplatte 34 angeordnet ist, verbunden. Die Anode 4 kann beispielsweise als Titanband ausgebildet sein. Die Referenzelektrode 6 misst das elektrische Potential im Bereich der Bewehrung 3 und kann somit die Funktion des Versorgungselektrodenpaares 5 überprüfen. Wie zuvor weisen die Kabelstränge 10 eine Versorgungsleitung 11 sowie eine von der Versorgungsleitung getrennte Datenleitung 12 auf.

Das Versorgungselektrodenpaar 5 ist mittels eines Wandlerknoten 29, der die für den kathodischen Korrosionsschutz notwendige Gleichspannung zur Verfügung stellt, mit dem Netzwerk 7 verbunden. Die Referenzelektrode 6 ist mittels eines Messknotens 31 mit dem Netzwerk 7 verbunden. Ein weiterer Messknoten 31 des Netzwerks 7 ist in diesem Beispiel mit einem Feuchtigkeitsmesser 36, der beispielsweise die Feuchtigkeit in der Wand 35 misst, verbunden. Die Daten der Messknoten 31 werden vorliegend über einen Aktivverteiler 22 und einen Passivverteiler 21 an die zentrale Schalteinheit 8 zurückgemeldet.

Die vorliegende Erfindung ist nicht auf die dargestellten und beschriebenen Ausführungsbeispiele beschränkt. Abwandlungen im Rahmen der Patentansprüche sind ebenso möglich wie eine Kombination der Merkmale, auch wenn diese in unterschiedlichen Ausführungsbeispielen dargestellt und beschrieben sind.

### Bezugszeichenliste

- 1: System
- 2: Bauwerk
- 3: Bewehrung
- 4: Anode
- 5: Versorgungselektrodenpaar
- 6: Referenzelektrode
- 7: Netzwerk
- 8: zentrale Schalteinheit
- 9: Verkabelung
- 10: Kabelstrang
- 11: Versorgungsleitung
- 12: Datenleitung
- 13: Ader
- 14: Ausgangsleitung
- 15: Zentralknoten
- 16: Computer
- 17: Netzteil
- 18: Modul zur kabellosen Datenübertragung
- 19: Powerlinemodul
- 20: Modem
- 21: Passivverteiler
- 22: Aktivverteiler
- 23: Eingangsleitung
- 24: externe Stromversorgung
- 25: zusätzliche Versorgungsleitung
- 26: Signalaufbereitungsmodul
- 27: Mikrocontroller
- 28: Speichermodul
- 29: Wandlerknoten
- 30: Spannungswandler
- 31: Messknoten
- 32: Analog-Digital-Wandler
- 33: Temperaturfühler
- 34: Bodenplatte
- 35: Wand
- 36: Feuchtigkeitsmesser

## Patentansprüche

1. System (1) für den kathodischen Korrosionsschutz eines Bauwerks (2) aus bewehrtem Beton mit
- wenigstens einem Versorgungselektrodenpaar (5) zum Beaufschlagen einer Bewehrung (3) des Bauwerks (2) mit einer Spannung,
- wenigstens einer Referenzelektrode (6) zur Messung eines elektrischen Potentials im Beton, wobei das Versorgungselektrodenpaar (5) und die Referenzelektrode (6) in einem Netzwerk (7) angeordnet sind, wobei das Netzwerk (7) weiterhin umfasst:
- eine zentrale Schalteinheit (8)
- eine Verkabelung (9) und
- wenigstens einen Verteiler (21, 22),
wobei die Verkabelung (9) wenigstens einen Kabelstrang (10) aufweist, **dadurch gekennzeichnet, dass** der Kabelstrang (10) eine Versorgungsleitung (11) und eine von der Versorgungsleitung (11) getrennte Datenleitung (12) umfasst und dass das Versorgungselektrodenpaar (5) über einen Wandlerknoten (29) mit dem Netzwerk (7) verbunden ist, wobei der Wandlerknoten wenigstens einen wenigstens zweistufigen Spannungswandler (30) aufweist.

2. System (1) gemäß dem vorangegangenen Anspruch, **dadurch gekennzeichnet, dass** die Versorgungsleitung (11) zwei Adern (13) und die Datenleitung (12) wenigstens zwei Adern (13) umfasst.

3. System (1) gemäß einem der vorangegangenen Ansprüche, **dadurch gekennzeichnet, dass** die Referenzelektrode (6) über einen Messknoten (31) mit dem Netzwerk (7) verbunden ist, wobei der Messknoten (31) wenigstens einen Analog-Digital-Wandler (32) und/oder Mikrocontroller (27) umfasst.

4. System (1) gemäß einem der vorangegangenen Ansprüche, **dadurch gekennzeichnet, dass** der Verteiler (21, 22) als Passivverteiler (21) oder Aktivverteiler (22) ausgebildet ist, wobei der Aktivverteiler (22) insbesondere eine externe Stromversorgung (24), eine mit der zentralen Schalteinheit verbundene zusätzliche Versorgungsleitung (25), ein Signalaufbereitungsmodul (26), einen Mikrocontroller (27) und/oder wenigstens zwei, vorzugsweise wenigstens vier, Ausgangsleitungen (14) aufweist.

5. System (1) gemäß einem der vorangegangenen Ansprüche, **dadurch gekennzeichnet, dass** die zentrale Schalteinheit (8) und/oder einer der wenigstens einen Aktivverteiler (22) ein Modul zur kabellosen Datenübertragung (18) aufweist.

6. System (1) gemäß einem der vorangegangenen Ansprüche, **dadurch gekennzeichnet, dass** das Modul zur kabellosen Datenübertragung (18) zur Verwendung von Kanalbreiten im Bereich von 180 kHz ausgebildet ist.

7. System (1) gemäß einem der vorangegangenen Ansprüche, **dadurch gekennzeichnet, dass** die zentrale Schalteinheit (8) und/oder einer der wenigstens einen Aktivverteiler (22) ein Powerlinemodul (19) zur Datenübertragung über das allgemeine Stromnetz aufweist.

8. System (1) gemäß einem der vorangegangenen Ansprüche, **dadurch gekennzeichnet, dass** die zentrale Schalteinheit (8) und/oder einer der wenigstens einen Aktivverteiler (22) ein Modem (20) zur Datenübertragung über das Internet aufweist.

9. System (1) gemäß einem der vorangegangenen Ansprüche, **dadurch gekennzeichnet, dass** der Wandlerknoten (29) wenigstens einen dreistufigen Spannungswandler (30) aufweist.

10. System (1) gemäß einem der vorangegangenen Ansprüche, **dadurch gekennzeichnet, dass** eine Gesamtanzahl der Passivverteiler (21), Wandlerknoten (29), Messknoten (31) und/oder Aktivverteiler (22) in dem Netzwerk (7) größer als 100 und insbesondere größer als 256 ist.

11. System (1) gemäß einem der vorangegangenen Ansprüche, **dadurch gekennzeichnet, dass** jeder Wandlerknoten (29), Messknoten (31) und/oder Aktivverteiler (22) ein Speichermodul (28) aufweist.

12. System (1) gemäß einem oder mehreren der Ansprüche 4 bis 11, **dadurch gekennzeichnet, dass** der Aktivverteiler (22) einen Mikrocontroller (27) und/oder wenigstens zwei, vorzugsweise wenigstens vier, Ausgangsleitungen (14) aufweist.

13. System (1) gemäß einem oder mehreren der Ansprüche 1 bis 11, mit einem ersten Spannungswandler (30), **dadurch gekennzeichnet, dass** der erste Spannungswandler (30) wenigstens zweistufig und vorzugsweise dreistufig ausgebildet ist.

## Claims

1. A system (1) for the cathodic corrosion protection of a structure (2) made of reinforced concrete, having
- at least one supply electrode pair (5) for applying a voltage to a reinforcement (3) of the structure (2),
- at least one reference electrode (6) for measuring an electrical potential in the concrete, the supply electrode pair (5) and the reference electrode (6) being disposed in a network (7), the network (7) further comprising:
- a central switching unit (8)
- a wiring (9) and
- at least one distributor (21, 22),
the wiring (9) having at least one cable harness (10), **characterized in that** the cable harness (10) comprises a supply line (11) and a data line (12) separate from the supply line (11) and that the supply electrode pair (5) is connected to the network (7) by means of a converter node (29), wherein the converter node comprises at least one at least two-stage voltage converter (30).

2. The system (1) according to the preceding claim, **characterized in that** the supply line (11) comprises two cores (13) and the data line (12) comprises at least two cores (13).

3. The system (1) according to any one of the preceding claims, **characterized in that** the reference electrode (6) is connected to the network (7) by means of a measuring node (31), wherein the measuring node (31) comprises at least one analogue/digital converter (32) and/or microcontroller (27).

4. The system (1) according to any one of the preceding claims, **characterized in that** the distributor (21, 22) is implemented as a passive distributor (21) or active distributor (22), wherein the active distributor (22) particularly comprises an external power supply (24), an additional supply line (25) connected to the central switching unit, a signal processing module (26), a microcontroller (27) and/or at least two, preferably at least four, output lines (14).

5. The system (1) according to any one of the preceding claims, **characterized in that** the central switching unit (8) and/or one of the at least one active distributor (22) comprises a module for wireless data transmission (18).

6. The system (1) according to any one of the preceding claims, **characterized in that** the module for wireless data transmission (18) is implemented for using channel widths in the range of 180 kHz.

7. The system (1) according to any one of the preceding claims, **characterized in that** the central switching unit (8) and/or one of the at least one active distributor (22) comprises a power line module (19) for data transmission by means of the general electricity network.

8. The system (1) according to any one of the preceding claims, **characterized in that** the central switching unit (8) and/or one of the at least one active distributor (22) comprises a modem (20) for data transmission by means of the Internet.

9. The system (1) according to any one of the preceding claims, **characterized in that** the converter node (29) comprises at least one three-stage voltage converter (30).

10. The system (1) according to any one of the preceding claims, **characterized in that** a total number of passive distributors (21), converter nodes (29), measuring nodes (31) and/or active distributors (22) in the network (7) is greater than 100 and particularly greater than 256.

11. The system (1) according to any one of the preceding claims, **characterized in that** each converter node (29), measuring node (31) and/or active distributor (22) comprises a memory module (28).

12. The system (1) according to any one or more of claims 4 to 11, **characterized in that** the active distributor (22) comprises a microcontroller (27) and/or at least two, preferably at least four, output lines (14).

13. The system (1) according to any one or more of claims 1 to 11, having a first voltage converter (30), **characterized in that** the first voltage converter (30) is implemented at least in two stages and preferably in three stages.

## Revendications

1. Système (1) pour la protection cathodique contre la corrosion d'un ouvrage (2) en béton armé, avec
- au moins une paire d'électrodes d'alimentation (5) pour appliquer une tension à une armature (3) de l'ouvrage (2),
- au moins une électrode de référence (6) pour mesurer un potentiel électrique dans le béton, dans lequel la paire d'électrodes d'alimentation (5) et l'électrode de référence (6) sont disposées dans un réseau (7), dans lequel le réseau (7) comprend en outre :
- une unité de commutation centrale (8),
- un câblage (9) et
- au moins un distributeur (21, 22),
dans lequel le câblage (9) présente au moins un faisceau de câbles (10), **caractérisé en ce que** le faisceau de câbles (10) comprend une ligne d'alimentation (11) et une ligne de données (12) séparée de la ligne d'alimentation (11) et **en ce que** la paire d'électrodes d'alimentation (5) est reliée au réseau (7) via un nœud de transformateur (29), dans lequel le nœud de transformateur présente au moins un transformateur de tension (30) à au moins deux niveaux.

2. Système (1) selon la revendication précédente, **caractérisé en ce que** la ligne d'alimentation (11) comprend deux conducteurs (13) et la ligne de données (12) comprend au moins deux conducteurs (13).

3. Système (1) selon l'une quelconque des revendications précédentes, **caractérisé en ce que** l'électrode de référence (6) est reliée au réseau (7) par l'intermédiaire d'un nœud de mesure (31), dans lequel le nœud de mesure (31) comprend au moins un convertisseur analogique-numérique (32) et/ou un microcontrôleur (27).

4. Système (1) selon l'une quelconque des revendications précédentes, **caractérisé en ce que** le distributeur (21, 22) est conçu en tant que distributeur passif (21) ou distributeur actif (22), dans lequel le distributeur actif (22) présente en particulier une alimentation électrique externe (24), une ligne d'alimentation supplémentaire (25) reliée à l'unité de commutation centrale, un module de conditionnement (26) de signal, un microcontrôleur (27) et/ou au moins deux, de préférence au moins quatre, lignes de sortie (14).

5. Système (1) selon l'une quelconque des revendications précédentes, **caractérisé en ce que** l'unité de commutation centrale (8) et/ou l'un des, au moins un, distributeurs actifs (22) comporte un module de transmission sans fil de données (18).

6. Système (1) selon l'une quelconque des revendications précédentes, **caractérisé en ce que** le module de transmission sans fil de données (18) est conçu pour utiliser des largeurs de canal de l'ordre de 180 kHz.

7. Système (1) selon l'une quelconque des revendications précédentes, **caractérisé en ce que** l'unité de commutation centrale (8) et/ou l'un des, au moins un, distributeurs actifs (22) comporte un module Powerline (19) pour la transmission de données via le réseau électrique général.

8. Système (1) selon l'une quelconque des revendications précédentes, **caractérisé en ce que** l'unité de commutation centrale (8) et/ou l'un des, au moins un, distributeurs actifs (22) comporte un modem (20) pour la transmission de données via Internet.

9. Système (1) selon l'une quelconque des revendications précédentes, **caractérisé en ce que** le nœud de transformateur (29) comporte au moins un transformateur de tension à trois niveaux (30).

10. Système (1) selon l'une quelconque des revendications précédentes, **caractérisé en ce qu'**un nombre total de distributeurs passifs (21), de nœud de transformateur (29), de nœuds de mesure (31) et/ou de distributeurs actifs (22) dans le réseau (7) est supérieur à 100 et en particulier supérieur à 256.

11. Système (1) selon l'une quelconque des revendications précédentes, **caractérisé en ce que** chaque nœud de transformateur (29), nœud de mesure (31) et/ou distributeur actif (22) comprend un module de mémoire (28).

12. Système (1) selon l'une quelconque ou plusieurs des revendications 4 à 11, **caractérisé en ce que** le distributeur actif (22) comprend un microcontrôleur (27) et/ou au moins deux, de préférence au moins quatre, lignes de sortie (14).

13. Système (1) selon l'une quelconque ou plusieurs des revendications 1 à 11, avec un premier transformateur de tension (30), **caractérisé en ce que** le premier transformateur de tension (30) est au moins à deux niveaux, et de préférence à trois niveaux.
